# EUROPEAN PATENT APPLICATION

(11) **EP 4 653 019 A1**
(43) Date of publication of application: **26.11.2025**
(21) Application number: 24744398.9
(22) Date of filing: 19.01.2024
(51) Int. Cl.: A61K 47/68, A61K 31/4745, A61K 31/519, A61K 31/565, A61K 39/395, A61P 35/00

(54) **ANTI-HER2 ANTIBODY-DRUG CONJUGATE FOR TREATING BREAST CANCER**

(30) Priority: 19.01.2023 CN 202310066299; 15.02.2023 CN 202310121195
(71) Applicant: Jiangsu Hengrui Pharmaceuticals Co., Ltd., Lianyungang, Jiangsu 222047 (CN); Shanghai Shengdi Pharmaceutical Co., Ltd, Shanghai 201210 (CN)
(72) Inventor: WANG, Quanren, Shanghai 201210 (CN)
(74) Representative: Regimbeau
(86) International application number: PCT/CN2024/073246
(87) International publication number: WO 2024/153229

(57) **Abstract**

Use of an anti-HER2 antibody-drug conjugate in the preparation of a drug for treating HER2 low-expression breast cancer. The structure of the antibody-drug conjugate is represented by formula (I).

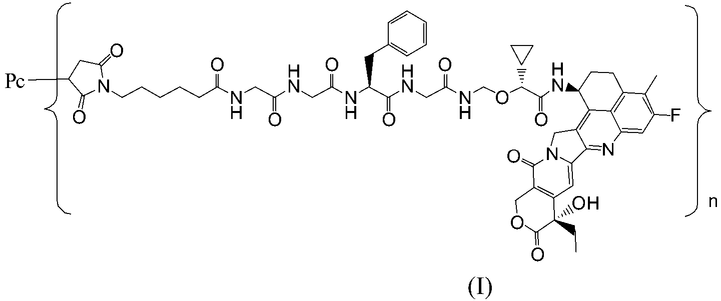

## Description

The present disclosure claims priority to Chinese Patent Application No. 202310066299X filed on January 19, 2023 and Chinese Patent Application No. 2023101211954 filed on February 15, 2023, which are incorporated herein by reference in their entirety.

### TECHNICAL FIELD

The present disclosure pertains to the field of pharmaceutics and relates to use of an anti-HER2 antibody-drug conjugate (ADC) in the manufacture of a medicament for treating breast cancer.

### BACKGROUND

Breast cancer is the most common malignancy worldwide. According to GLOBOCAN2020 statistics, breast cancer ranked first among malignancies in women in terms of both morbidity and mortality. There are about 2.26 million new cases of breast cancer and 685,000 deaths from it every year worldwide. Breast cancer ranks first among malignancies in women in terms of morbidity and mortality.

In clinical practice, about 55% of breast cancer patients have low HER2 expression. Low HER2 expression refers to an immunohistochemistry (IHC) score of 1+ or IHC 2+ and *in situ* hybridization (ISH)-. Existing HER2-targeting drugs fail to show significant benefits. There is a huge unmet clinical need regarding metastatic breast cancer with low HER2 expression. Firstly, for patients with low HER2 expression, the prior treatment is equivalent to that for HER2-negative patients, and the options for advanced treatment are limited. Secondly, for HR+/HER2- patients who developed resistance after receiving treatment with CDK4/6 inhibitors or endocrine therapy and experienced disease progression, the subsequent options are limited.

HER2, a member of the type I transmembrane tyrosine kinase receptor family, is substantially inactive in its monomeric state, but can polymerize with the other 3 transmembrane tyrosine kinase members of the family, HER1, HER3, and HER4, resulting in phosphorylation of receptor tyrosine residues and initiation of various signaling pathways (e.g., MAPK and PI3K/Akt), thereby promoting cell proliferation, tumorigenesis, and progression.

An ADC targeting HER2 acts in the following way: first, the antibody in the ADC specifically recognizes and binds to the HER2 receptor on the surface of a target cell, then the ADC enters the target cell via endocytosis and is decomposed in the cell to release a cytotoxic drug, and finally, the cytotoxic drug induces apoptosis of the cell by damaging DNA or acting on tubulin, thereby exerting an anti-tumor effect. If the cytotoxic drug has high membrane permeability, after being released in the target cell, it can penetrate into the extracellular space and kill the surrounding HER2-negative cells. This effect is referred to as a bystander killing effect. This effect may also occur if the cytotoxic drug is released before endocytosis of the ADC. WO2020063676A discloses a class of ADCs targeting HER2. In view of the excellent effects of the approved HER-2 ADC drugs trastuzumab emtansine (TDM-1) and trastuzumab deruxtecan (DS-8201) in the treatment of breast cancer and gastric cancer, it is of great significance to study indications for the antibody-drug conjugates in WO2020063676A.

In summary, there is an urgent need to provide safer and more effective clinical treatment regimens for breast cancer patients with low HER2 expression.

### SUMMARY

The present disclosure provides pharmaceutical use and a method of an anti-HER2 antibody-drug conjugate for treating a tumor.

In some embodiments, the present disclosure provides any one of the following uses or methods of the anti-HER2 antibody-drug conjugate:
(1) use of the anti-HER2 antibody-drug conjugate in the manufacture of a medicament for treating breast cancer with low HER2 expression,
(2) the anti-HER2 antibody-drug conjugate for use in treating breast cancer with low HER2 expression, and
(3) a method for treating breast cancer with low HER2 expression, comprising administering the anti-HER2 antibody-drug conjugate to a subject in need thereof.

In some embodiments, the antibody-drug conjugate has a structure represented by formula (I): 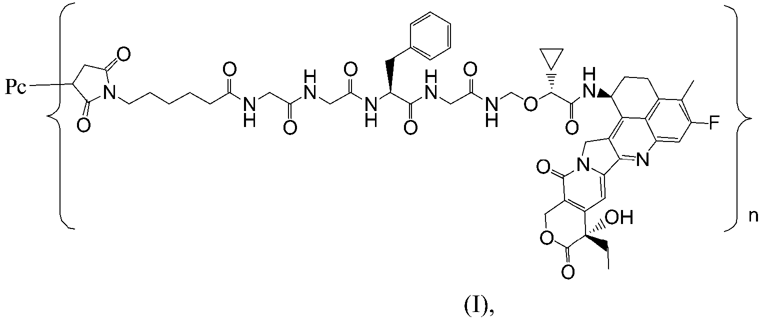 wherein:
n is a decimal or integer from 3 to 8; for example, n is 3, 4, 5, 6, 7, 8, or any integer or decimal between any two of the aforementioned numerical values.

Pc is an anti-HER2 antibody.

In the present disclosure, the term "antibody" is used in the broadest sense and encompasses a variety of antibody structures, including but not limited to monoclonal antibodies, polyclonal antibodies, multispecific antibodies (e.g., bispecific antibodies), full-length antibodies, or antigen-binding fragments thereof (also known as "antigen-binding moieties") so long as they exhibit the desired antigen-binding activity.

In some embodiments, the anti-HER2 antibody or the antigen-binding fragment thereof described in the present disclosure is selected from the group consisting of trastuzumab or an antigen-binding fragment thereof and pertuzumab or an antigen-binding fragment thereof.

In some embodiments, the anti-HER2 antibody or the antigen-binding fragment thereof described in the present disclosure is trastuzumab or an antigen-binding fragment thereof.

In some embodiments, the anti-HER2 antibody comprises a heavy chain variable region (VH) and a light chain variable region (VL), wherein the heavy chain variable region comprises a HCDR1, a HCDR2, and a HCDR3 set forth in SEQ ID NO: 1, SEQ ID NO: 2, and SEQ ID NO: 3, respectively, and the light chain variable region comprises a LCDR1, a LCDR2, and a LCDR3 set forth in SEQ ID NO: 4, SEQ ID NO: 5, and SEQ ID NO: 6, respectively.

The aforementioned CDR sequences are shown in Table 1 below:

| Antibody | Anti-HER2 antibody |
|---|---|
| HCDR1 | DTYIH (SEQ ID NO: 3) |
| HCDR2 | RIYPTNGYTRYADSVKG (SEQ ID NO: 4) |
| HCDR3 | WGGDGFYAMDY (SEQ ID NO: 5) |
| LCDR1 | RASQDVNTAVA (SEQ ID NO: 6) |
| LCDR2 | SASFLYS (SEQ ID NO: 7) |
| LCDR3 | QQHYTTPPT (SEQ ID NO: 8) |

In some specific embodiments, the CDRs are defined according to the Kabat numbering scheme.

In some embodiments, the anti-HER2 antibody comprises any 1, 2, 3, 4, 5, or 6 of the aforementioned HCDR1, HCDR2, HCDR3, LCDR1, LCDR2, and LCDR3.

In some embodiments, the anti-HER2 antibody is a humanized antibody. In some specific embodiments, the heavy chain variable region comprises the amino acid sequence set forth in SEQ ID NO: 9 or an amino acid sequence having at least 80% sequence identity thereto, and the light chain variable region comprises the amino acid sequence set forth in SEQ ID NO: 10 or an amino acid sequence having at least 80% sequence identity thereto.

Heavy chain variable region:




SEQ ID NO: 9;

Light chain variable region:



SEQ ID NO: 10;

In some embodiments, the anti-HER2 antibody comprises any one of or a combination of any two of the aforementioned VH and VL.

In some embodiments, the anti-HER2 antibody further comprises a heavy chain constant region and/or a light chain constant region. Illustratively, the light/heavy chain constant regions described above are combined with the variable regions of the aforementioned antibody to form a complete antibody whose light/heavy chain sequences are as follows:
Heavy chain: 
EVQLVESGGGLVQPGGSLRLSCAASGFNIKDTYIHWVRQAPGKGLEWVARIYPTNGYT
RYADSVKGRFTISADTSKNTAYLQMNSLRAEDTAVYYCSRWGGDGFYAMDYWGQGT
LVTVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPELLGG
PSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSREEMTK
NQVSLTCLVKGFYPSDIAVEWESNGQPENNYKTTPPVLDSDGSFFLYSKLTVDKSRWQQGN
 
VFSCSVMHEALHNHYTQKSLSLSPGK
SEQ ID NO: 2
Light chain:

DIQMTQSPSSLSASVGDRVTITCRASQDVNTAVAWYQQKPGKAPKLLIYSASFLYSGVP
SRFSGSRSGTDFTLTISSLQPEDFATYYCQQHYTTPPTFGQGTKVEIKRTVAAPSVFIFPPS
DEQLKSGTASVVCLLNNFYPREAKVQWKVDNALQSGNSQESVTEQDSKDSTYSLSSTLTLSK
ADYEKHKVYACEVTHQGLSSPVTKSFNRGEC
SEQ ID NO: 1

In some embodiments, the heavy chain of the anti-HER2 antibody comprises the amino acid sequence set forth in SEQ ID NO: 2 or an amino acid sequence having at least 80% sequence identity thereto, and the light chain of the anti-HER2 antibody comprises the amino acid sequence set forth in SEQ ID NO: 1 or an amino acid sequence having at least 80% sequence identity thereto.

In some embodiments, the anti-HER2 antibody provided in the present disclosure comprises any one of or a combination of any two of the aforementioned heavy and light chains.

In the context of the present disclosure, "at least 80%" encompasses 80% or more, e.g., at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, and a numerical range between any two of these numbers.

In some embodiments, the anti-HER2 antibody-drug conjugate described above may be prepared by referring to the method in WO2021190581A. The content regarding the ADC structures, antibody sequences, and preparation methods in WO2021190581A is incorporated herein by reference.

In some embodiments, the anti-HER2 antibody-drug conjugate described in the present disclosure has a structure represented by the following formula: 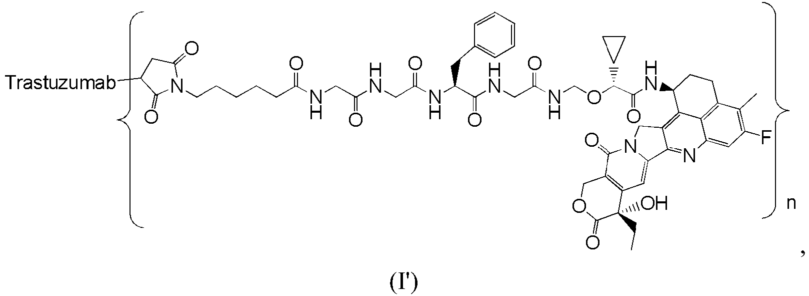 wherein n is a decimal or integer from 3 to 8.

In some embodiments, n is 6 ± 0.8. For example, n is 5.2, 5.3, 5.4, 5.5, 5.6, 5.7, 5.8, 5.9, 6.0, 6.1, 6.2, 6.3, 6.4, 6.5, 6.6, 6.7, or 6.8.

In an optional embodiment, in the anti-HER2 antibody-drug conjugate described in the present disclosure, n is 6.

In the present disclosure, the "breast cancer with low HER2 expression" is not particularly limited so long as it is considered to be breast cancer with low HER2 expression by those skilled in the art. Preferred examples of breast cancer with low HER2 expression may include IHC2+ and ISH negative, IHC1+ and ISH negative, IHC1+ and ISH not tested, 0 < IHC < 1+.

In some embodiments, the breast cancer with low HER2 expression is breast cancer with HER2 expression determined to be 1+ by immunohistochemistry, i.e., IHC1+, e.g., IHC1+/ISH- or IHC1+/ISH not tested.

In some embodiments, the breast cancer with low HER2 expression is breast cancer with HER2 expression determined to be 2+ by immunohistochemistry and to be negative by *in situ* hybridization, i.e., IHC2+/ISH-.

In some embodiments, the breast cancer with low HER2 expression is unresectable, recurrent, and/or metastatic breast cancer with low HER2 expression.

In some embodiments, the breast cancer with low HER2 expression is unresectable or metastatic breast cancer with low HER2 expression.

In some embodiments, the breast cancer with low HER2 expression is a patient with recurrent or metastatic breast cancer with low HER2 expression.

In some embodiments, the breast cancer patient is an HR-positive patient or an HR-negative patient.

In some embodiments, the breast cancer patient has received at least first-line endocrine therapy.

In some embodiments, the breast cancer patient has not previously received or has previously received chemotherapy.

In some embodiments, the breast cancer patient with low HER2 expression has previously received treatment with an anti-HER2 drug. In some embodiments, the breast cancer patient with low HER2 expression develops resistance or refractoriness after previously receiving treatment with an anti-HER2 drug.

In the present disclosure, "resistance" or "refractoriness" refers to the property of not responding to anti-cancer agent-based treatment and may also be expressed as "non-responsiveness".

In some embodiments, the anti-HER2 antibody-drug conjugate provided in the present disclosure is used for treating a breast cancer patient with low HER2 expression and can significantly improve the patient's tumor objective response rate and progression-free survival. Particularly, at a dose of 6.4 mg/kg, the conjugate can achieve a cORR of 64.9% and an mPFS of 13.8 months. The conjugate has significant therapeutic advantages over other ADC drugs for the same target and provides a positive and effective clinical treatment regimen for patients with breast cancer with low HER2 expression.

In some embodiments, the anti-HER2 antibody-drug conjugate provided in the present disclosure, when administered to a subject, can significantly reduce the incidence of interstitial pneumonia, has improved clinical safety, and is beneficial for expanding the clinical administration window.

In some embodiments, the anti-HER2 antibody-drug conjugate is used in combination with a second therapeutic agent.

In some embodiments, the second therapeutic agent is one agent or a combination of two or more agents selected from the group consisting of a CDK4/6 inhibitor, a SERD, a VEGF ligand inhibitor, an aromatase inhibitor, and a CDK4/6 inhibitor.

In some embodiments, the anti-HER2 antibody-drug conjugate is used in combination with a CDK4/6 inhibitor.

In some embodiments, the anti-HER2 antibody-drug conjugate is used in combination with a SERD (selective estrogen receptor degrader).

In some embodiments, the anti-HER2 antibody-drug conjugate is used in combination with a VEGF ligand inhibitor.

In some embodiments, the anti-HER2 antibody-drug conjugate is used in combination with an aromatase inhibitor.

In some embodiments, the anti-HER2 antibody-drug conjugate is used in combination with a CDK4/6 inhibitor and an aromatase inhibitor.

In some embodiments, the CDK4/6 inhibitor described in the present disclosure is selected from the group consisting of abemaciclib, ribociclib, palbociclib, alvocidib, trilaciclib, voruciclib, AT-7519, G1T-38, FLX-925, INOC-005, G1T28-1, BPI-1178, gossypin, G1T30-1, GZ-38-1, P-276-00, staurosporine, R-547, PAN-1215, PD-0183812, AG-024322, NSC-625987, CGP-82996, PD-171851, and the compound represented by formula (II) or a pharmaceutically acceptable salt thereof. In some embodiments, the CDK4/6 inhibitor is the compound represented by formula (II) or a pharmaceutically acceptable salt thereof:

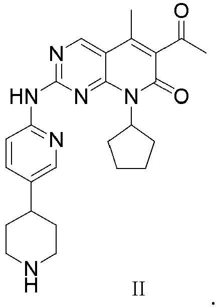

In some embodiments, the pharmaceutically acceptable salt of the compound represented by formula II is isethionate.

In some embodiments, the SERD described in the present disclosure is selected from the group consisting of fulvestrant, AZD-9496, RAD1901, and ZB-716. In some embodiments, the SERD is fulvestrant.

In some embodiments, the VEGF ligand inhibitor described in the present disclosure is selected from the group consisting of bevacizumab, ramucirumab, ranibizumab, aflibercept, conbercept, abicipar pegol, brolucizumab, LMG-324, nesvacumab, sevacizumab, tanibirumab, navicixizumab, RG-7716, LHA-510, OPT-302, TK-001, GZ-402663, VGX-100, PG-545, BI-836880, GNR-011, BR-55, OTSGC-A24, PAN-90806, AVA-101, ODM-203, TAS-115, X-82, MP-0250, sitravatinib, 4SC-203, AL-2846, ABT-165, SIM-010603, BI-836880, HL-217, CS-2164, RGX-314, AMC-303, and VXM-01. In some embodiments, the VEGF ligand inhibitor is bevacizumab.

In some embodiments, the aromatase inhibitor described in the present disclosure is selected from the group consisting of formestane, exemestane, fadrozole, letrozole, vorozole, and anastrozole. In some embodiments, the aromatase inhibitor is letrozole or anastrozole.

In some embodiments, the anti-HER2 antibody-drug conjugate described in the present disclosure is administered at a dose of 1.0 mg/kg-10.0 mg/kg. In an optional embodiment, the anti-HER2 antibody-drug conjugate described in the present disclosure is administered at doses of 1.0 mg/kg, 1.2 mg/kg, 1.4 mg/kg, 1.6 mg/kg, 1.8 mg/kg, 2.0 mg/kg, 2.2 mg/kg, 2.4 mg/kg, 2.6 mg/kg, 2.8 mg/kg, 3.0 mg/kg, 3.2 mg/kg, 3.4 mg/kg, 3.6 mg/kg, 3.8 mg/kg, 4.0 mg/kg, 4.2 mg/kg, 4.4 mg/kg, 4.6 mg/kg, 4.8 mg/kg, 5.0 mg/kg, 5.2 mg/kg, 5.4 mg/kg, 5.6 mg/kg, 5.8 mg/kg, 6.0 mg/kg, 6.2 mg/kg, 6.4 mg/kg, 6.6 mg/kg, 6.8 mg/kg, 7.0 mg/kg, 7.2 mg/kg, 7.4 mg/kg, 7.6 mg/kg, 7.8 mg/kg, 8.0 mg/kg, 8.2 mg/kg, 8.4 mg/kg, 8.6 mg/kg, 8.8 mg/kg, 9.0 mg/kg, 9.2 mg/kg, 9.4 mg/kg, 9.6 mg/kg, 9.8 mg/kg, or 10.0 mg/kg. In an optional embodiment, the anti-HER2 antibody-drug conjugate described in the present disclosure is administered at a dose of 1.0 mg/kg, 2.0 mg/kg, 3.2 mg/kg, 4.8 mg/kg, 5.6 mg/kg, 6.4 mg/kg, or 8.0 mg/kg.

In some embodiments, the anti-HER2 antibody-drug conjugate described in the present disclosure is administered at a frequency of at least once every week, at least once every two weeks, at least once every three weeks, at least once every four weeks, or at least once every six weeks. In an optional embodiment, the frequency of administration is once every week, once every two weeks, once every three weeks, or once every four weeks. In an optional embodiment, the frequency of administration is once every two weeks or once every three weeks.

In an optional embodiment, the anti-HER2 antibody-drug conjugate described in the present disclosure is administered at a dose of 1.0 mg/kg, 2.0 mg/kg, 3.2 mg/kg, 4.8 mg/kg, 5.6 mg/kg, 6.4 mg/kg, or 8.0 mg/kg and at a frequency of once every two weeks or once every three weeks.

In some embodiments, the CDK4/6 inhibitor described in the present disclosure is administered at a dose of 1-1000 mg. In an optional embodiment, the CDK4/6 inhibitor described in the present disclosure may be administered at a dose of 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 100 mg, 125 mg, 150 mg, 175 mg, 200 mg, 250 mg, 300 mg, 350 mg, 400 mg, 450 mg, 500 mg, 600 mg, 700 mg, 750 mg, 800 mg, 900 mg, or 1000 mg. In an optional embodiment, the dose is 100 mg, 125 mg, or 150 mg.

The frequency of administration may be once daily, twice daily, three times daily, once weekly, once every two weeks, once every three weeks, or once monthly. In an optional embodiment, the frequency of administration is once daily.

In an optional embodiment, the CDK4/6 inhibitor described in the present disclosure is administered at a dose of 75 mg, 100 mg, 125 mg, or 150 mg and at a frequency of once daily.

In some embodiments, the SERD described in the present disclosure is administered at a dose of 1-1000 mg. In an optional embodiment, the SERD described in the present disclosure may be administered at a dose of 5 mg, 10 mg, 12.5 mg, 15 mg, 17.5 mg, 20 mg, 25 mg, 30 mg, 35 mg, 40 mg, 45 mg, 50 mg, 60 mg, 70 mg, 75 mg, 80 mg, 90 mg, 100 mg, 125 mg, 150 mg, 175 mg, 200 mg, 225 mg, 250 mg, 275 mg, 300 mg, 325 mg, 350 mg, 375 mg, 400 mg, 425 mg, 450 mg, 475 mg, 500 mg, 600 mg, 700 mg, 750 mg, 800 mg, 900 mg, or 1000 mg. In an optional embodiment, the SERD described in the present disclosure is administered at a dose of 500 mg.

In some embodiments, the SERD described in the present disclosure may be administered at a frequency of once daily, twice daily, three times daily, once weekly, once every two weeks, once every three weeks, or once monthly. In some embodiments, the frequency of administration may be once daily, once weekly, once every two weeks, once every three weeks, or once monthly, e.g., once every two weeks or once monthly.

In an optional embodiment, the SERD described in the present disclosure is administered at a dose of 500 mg and at a frequency of once every two weeks or once monthly.

In some embodiments, the VEGF ligand inhibitor described in the present disclosure is administered at a dose of 0.1-100 mg/kg. In an optional embodiment, the VEGF ligand inhibitor described in the present disclosure may be administered at a dose of 0.5 mg/kg, 1 mg/kg, 2 mg/kg, 2.5 mg/kg, 3 mg/kg, 4 mg/kg, 5 mg/kg, 6 mg/kg, 7 mg/kg, 7.5 mg/kg, 8 mg/kg, 9 mg/kg, 10 mg/kg, 12.5 mg/kg, 12 mg/kg, 15 mg/kg, 17.5 mg/kg, 20 mg/kg, 25 mg/kg, or 30 mg/kg. In an optional embodiment, the VEGF ligand inhibitor described in the present disclosure is administered at a dose of 15 mg/kg.

In some embodiments, the VEGF ligand inhibitor described in the present disclosure may be administered at a frequency of once daily, once weekly, once every two weeks, once every three weeks, or once monthly. In an optional embodiment, the frequency of administration may be once weekly, once every two weeks, once every three weeks, or once monthly. In an optional embodiment, the frequency of administration is once every three weeks.

In an optional embodiment, the VEGF ligand inhibitor described in the present disclosure is administered at a dose of 15 mg/kg and at a frequency of once every three weeks.

In some embodiments, the aromatase inhibitor described in the present disclosure is administered at a dose of 0.1-50 mg. In an optional embodiment, the aromatase inhibitor described in the present disclosure may be administered at a dose of 0.1 mg, 0.25 mg, 0.5 mg, 0.75 mg, 1 mg, 1.25 mg, 1.5 mg, 1.75 mg, 2 mg, 2.25 mg, 2.5 mg, 2.75 mg, 3 mg, 3.25 mg, 3.5 mg, 3.75 mg, 4 mg, 4.25 mg, 4.5 mg, 4.75 mg, 5 mg, 5.5 mg, 6 mg, 6.5 mg, 7 mg, 7.5 mg, 8 mg, 8.5 mg, 9 mg, 9.5 mg, 10 mg, 12.5 mg, 15 mg, 17.5 mg, 20 mg, 25 mg, 30 mg, 35 mg, 40 mg, 45 mg, or 50 mg. In an optional embodiment, the aromatase inhibitor described in the present disclosure is administered at a dose of 0.5 mg, 1.0 mg, 1.5 mg, 2.0 mg, 2.5 mg, 3.0 mg, 3.5 mg, 4.0 mg, 4.5 mg, or 5.0 mg.

In an optional embodiment, the aromatase inhibitor described in the present disclosure is administered at a frequency of once daily or twice daily. In an optional embodiment, the frequency of administration is once daily or twice daily.

In an optional embodiment, the aromatase inhibitor described in the present disclosure is administered at a dose of 0.5 mg, 1.0 mg, 1.5 mg, 2.0 mg, 2.5 mg, 3.0 mg, 3.5 mg, 4.0 mg, 4.5 mg, or 5.0 mg and at a frequency of once daily or twice daily.

In an optional embodiment, the aromatase inhibitor described in the present disclosure is letrozole and is administered at a dose of 2.5 mg and at a frequency of once daily.

In an optional embodiment, the aromatase inhibitor described in the present disclosure is anastrozole and is administered at a dose of 1 mg and at a frequency of once daily.

In some embodiments, the anti-HER2 antibody-drug conjugate described in the present disclosure is administered once every two weeks or once every three weeks; the CDK4/6 inhibitor described in the present disclosure is administered once daily, with continuous administration for the first 2 weeks (days 1 to 14) and interruption (no administration) for the following week (days 15 to 21), or continuous administration for the first 3 weeks and interruption for the following week.

In an optional embodiment, the anti-HER2 antibody-drug conjugate described in the present disclosure is administered once every two weeks or once every three weeks; the SERD described in the present disclosure is administered with every 4 weeks as one treatment cycle, wherein administration is performed on days 1 and 15 of the first cycle and day 1 of every subsequent cycle.

In an optional embodiment, the anti-HER2 antibody-drug conjugate described in the present disclosure is administered once every two weeks or once every three weeks; the VEGF ligand inhibitor described in the present disclosure is administered once every three weeks.

In an optional embodiment, the anti-HER2 antibody-drug conjugate described in the present disclosure is administered once every two weeks or once every three weeks; the aromatase inhibitor described in the present disclosure is continuously administered once daily.

In an optional embodiment, the anti-HER2 antibody-drug conjugate described in the present disclosure is administered once every two weeks or once every three weeks; the CDK4/6 inhibitor described in the present disclosure is administered once daily, with continuous administration for the first 2 weeks (days 1 to 14) and interruption (no administration) for the following week (days 15 to 21), or continuous administration for the first 3 weeks and interruption for the following week; the aromatase inhibitor described in the present disclosure is continuously administered once daily.

In some embodiments, the treatment cycle is one treatment cycle every two weeks, one treatment cycle every three weeks, or one treatment cycle every four weeks.

The present disclosure further provides use of an anti-HER2 antibody-drug conjugate in combination with a second therapeutic agent in the manufacture of a medicament for treating breast cancer, wherein the antibody-drug conjugate has a structure represented by formula (I): 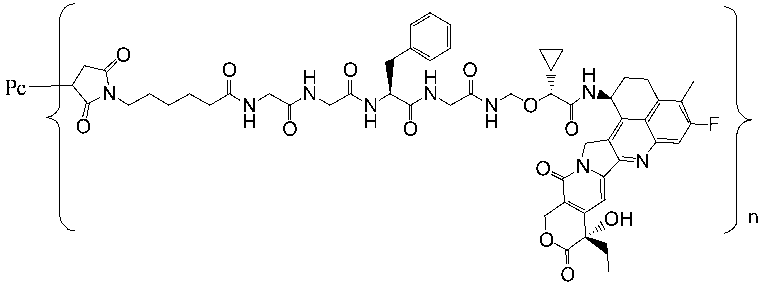 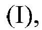 wherein:
n is a decimal or integer from 3 to 8;
Pc is an anti-HER2 antibody or an antigen-binding fragment thereof;
the second therapeutic agent is one or more agents selected from the group consisting of a CDK4/6 inhibitor, a SERD, a VEGF ligand inhibitor, and an aromatase inhibitor.

In some embodiments, the anti-HER2 antibody-drug conjugate is used in combination with a CDK4/6 inhibitor.

In some embodiments, the anti-HER2 antibody-drug conjugate is used in combination with a SERD.

In some embodiments, the anti-HER2 antibody-drug conjugate is used in combination with a VEGF ligand inhibitor.

In some embodiments, the anti-HER2 antibody-drug conjugate is used in combination with an aromatase inhibitor.

In some embodiments, the anti-HER2 antibody-drug conjugate is used in combination with a CDK4/6 inhibitor and an aromatase inhibitor.

In some embodiments, the breast cancer is breast cancer with low HER2 expression.

In some embodiments, the breast cancer with low HER2 expression is breast cancer with HER2 expression determined to be 1+ by immunohistochemistry, i.e., IHC1+, e.g., IHC1+/ISH- or IHC1+/ISH not tested.

In some embodiments, the breast cancer with low HER2 expression is breast cancer with HER2 expression determined to be 2+ by immunohistochemistry and to be negative by *in situ* hybridization, i.e., IHC2+/ISH-.

In some embodiments, the breast cancer with low HER2 expression is unresectable, recurrent, and/or metastatic breast cancer with low HER2 expression.

In an optional embodiment, the breast cancer is unresectable or metastatic breast cancer.

In an optional embodiment, the breast cancer patient is an HR-positive patient or an HR-negative patient.

In an optional embodiment, the breast cancer patient has received at least first-line endocrine therapy.

In an optional embodiment, the breast cancer patient has not previously received or has previously received chemotherapy.

In some embodiments, the treatment cycle of the present disclosure is one treatment cycle every two weeks, one treatment cycle every three weeks, or one treatment cycle every four weeks, e.g., one treatment cycle every three weeks.

In another aspect, the present disclosure provides a pharmaceutical composition comprising the aforementioned anti-HER2 antibody-drug conjugate and a second therapeutic agent, and one or more pharmaceutically acceptable carriers, wherein the second therapeutic agent is one or more agents selected from the group consisting of a CDK4/6 inhibitor, a SERD, a VEGF ligand inhibitor, and an aromatase inhibitor.

In another aspect, the present disclosure provides the aforementioned anti-HER2 antibody-drug conjugate for use in treating breast cancer with low HER2 expression.

In another aspect, the present disclosure provides the aforementioned anti-HER2 antibody-drug conjugate for use in treating breast cancer, wherein the anti-HER2 antibody-drug conjugate is used in combination with the aforementioned therapeutic agent.

The pharmaceutical composition, use, and treatment method of the present disclosure may also be used as adjuvant chemotherapy in combination with a surgical procedure. The treatment method of the present disclosure may be administered before the surgical procedure, aiming to reduce tumor size (referred to as preoperative adjuvant chemotherapy or neoadjuvant therapy), or may be administered after the surgical procedure, aiming to prevent tumor recurrence (referred to as postoperative adjuvant chemotherapy or adjuvant therapy).

The method of scoring the degree of HER2 expression by immunohistochemistry or the method of determining the positivity or negativity for HER2 expression by *in situ* hybridization is not particularly limited so long as it is recognized by those skilled in the art.

### Terminology

In order to facilitate the understanding of the present disclosure, certain technical and scientific terms are specifically defined below. Unless otherwise specifically defined herein, all other technical and scientific terms used herein have the meanings generally understood by those of ordinary skill in the art to which the present disclosure pertains.

Application No. WO2020063676A is incorporated herein by reference in its entirety.

"Antibody-drug conjugate (ADC)" is formed by connecting an antibody or an antibody fragment to a cytotoxin with biological activity or a small-molecule drug with cell-killing activity via a stable chemical linker compound, in which case the binding specificity of the antibody for tumor cell-specific or highly expressed antigens and the high efficiency of the cytotoxin are fully exploited and toxic side effects on normal cells are avoided. Antibody-drug conjugates can bind to tumor cells precisely and reduce the impact on normal cells compared with conventional chemotherapeutic drugs.

If an antibody-drug conjugate shows no significant chemical change, then the antibody "retains its chemical stability" in a pharmaceutical formulation. Chemical stability can be evaluated by detecting and quantifying chemically changed proteins. Degradation processes that often change the chemical structure of proteins include hydrolysis or clipping (evaluated by methods such as size exclusion chromatography and CE-SDS), oxidation (evaluated by methods such as peptide mapping in conjunction with mass spectroscopy or MALDI/TOF/MS), deamidation (evaluated by methods such as ion-exchange chromatography, capillary isoelectric focusing, peptide mapping, and isoaspartic acid measurement), and isomerization (evaluated by measuring the isoaspartic acid content, peptide mapping, etc.).

An antibody-drug conjugate "retains its biological activity" in a pharmaceutical formulation if the biological activity of the antibody-drug conjugate at a given time is within a predetermined range of the biological activity exhibited at the time the pharmaceutical formulation was prepared.

The term "antibody" described in the present disclosure is used in the broadest sense and encompasses a variety of antibody structures, including but not limited to monoclonal antibodies, polyclonal antibodies; monospecific antibodies, multispecific antibodies (e.g., bispecific antibodies), full-length antibodies, and antibody fragments (or antigen-binding fragments, or antigen-binding moieties), so long as they exhibit the desired antigen-binding activity. For example, an antibody may refer to an immunoglobulin, which is an intact antibody, a four-peptide-chain structure formed by linking two identical heavy chains and two identical light chains by interchain disulfide bonds. The heavy chain constant regions of an immunoglobulin differ in their amino acid composition and arrangement, and thus in their antigenicity. Accordingly, immunoglobulins can be divided into five classes, otherwise called isotypes of immunoglobulins, namely IgM, IgD, IgG, IgA, and IgE, with their corresponding heavy chains being µ chain, δ chain, γ chain, α chain, and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3, and IgG4. Light chains are divided into κ or λ chains according to differences in the constant regions. Each of the five classes of Ig may have a κ chain or λ chain.

The term "antibody or antigen-binding thereof" or "functional fragment" described in the present disclosure refers to Fab fragments, Fab' fragments, and F(ab')2 fragments that have antigen-binding activity, and Fv fragments and scFv fragments that bind to antibodies. An Fv fragment comprises an antibody heavy chain variable region and an antibody light chain variable region but does not comprise a constant region, and has the smallest antibody fragment of all antigen-binding sites. Generally, an Fv antibody also comprises a polypeptide linker between the VH and VL domains, and is capable of forming a structure required for antigen binding. Two antibody variable regions can also be linked using different linkers to form a single polypeptide chain known as a single-chain antibody or single-chain Fv (sFv).

The term "linker unit" or "linker fragment" refers to a chemical structure fragment or bond that is linked to an antibody or an antigen-binding fragment thereof at one end and to a drug at the other end, and it may also be linked to a drug after being linked to another linker. In a preferred embodiment of the present disclosure, they are represented as L and L¹ through L⁴, wherein the L¹ end is linked to an antibody, and the L⁴ end is linked to structure unit Y and then to a compound or toxin.

Linkers include stretcher units, spacer units, and amino acid units, and may be synthesized by methods known in the art, such as those described in US2005-0238649A1. A linker may be a "cleavable linker" that facilitates the release of drugs in cells. For example, acid-labile linkers (e.g., hydrazones), protease-sensitive (e.g., peptidase-sensitive) linkers, photolabile linkers, dimethyl linkers, or disulfide-containing linkers may be used (Chari et al., Cancer Research, 52: 127-131(1992); U.S. Patent No. 5,208,020).

The term "drug loading" refers to the mean number of cytotoxic drugs loaded on each antibody or an antigen-binding fragment thereof in the molecule of formula (I), and it may also be expressed as the ratio of the number of drugs to the number of antibodies. The drug loading may range from 0-12, preferably 1-10, more preferably 3-8, and most preferably 5.3-6.1 cytotoxic drugs (D) linked to each antibody or an antigen-binding fragment (Pc) thereof. In an embodiment of the present disclosure, the drug loading is represented as n; illustratively, the drug loading may be an average of 1, 2, 3, 4, 5, 6, 7, 8, 9, or 10. The average number of drugs per ADC molecule after a coupling reaction can be characteristically identified by conventional methods such as UV/visible spectroscopy, mass spectrometry, ELISA, and HPLC.

In one embodiment of the present disclosure, the cytotoxic drug is coupled to the N-terminal amino of the antibody or the antigen-binding fragment thereof, the ε-amino group and/or the sulfhydryl group of a lysine residue through a linker unit, and generally, the number of drug molecules that can be coupled to the antibody in a coupling reaction will be less than the theoretical maximum.

The loading of the cytotoxic drug can be controlled using the following non-limiting methods, including:
(1) controlling a molar ratio of a linking reagent to a monoclonal antibody,
(2) controlling reaction time and temperature, and
(3) selecting different reaction reagents.

For the preparation of conventional pharmaceutical compositions, refer to Chinese Pharmacopoeia.

"Administer" and "treat", when applied to animals, humans, experimental subjects, cells, tissues, organs, or biological fluids, refer to contact of an exogenous drug, a therapeutic agent, a diagnostic agent, or a composition with the animals, humans, subjects, cells, tissues, organs, or biological fluids. "Administer" and "treat" can refer to, for example, therapeutic, pharmacokinetic, diagnostic, research, and experimental methods. The treatment of cells comprises contacting a reagent with cells and contacting the reagent with a fluid, where the fluid is in contact with the cells. "Administer" and "treat" also refer to treating, e.g., cells, by reagents, diagnosis, binding compositions or by another cell *in vitro* and *ex vivo.* "Treating", when applied to humans, veterinary or research subjects, refers to therapeutic treatment, preventive or prophylactic measures, and research and diagnostic applications.

"Treatment" refers to administering a therapeutic agent, such as a composition comprising any one of the conjugation compounds of the present disclosure, either internally or externally to a patient with one or more symptoms of a disease on which the therapeutic agent is known to have a therapeutic effect. Typically, the therapeutic agent is administered in an amount effective to alleviate one or more disease symptoms in the patient or population being treated to induce regression of such symptoms or to inhibit the development of such symptoms to any clinically measurable degree. The amount of the therapeutic agent effective to alleviate any specific disease symptom (also known as a "therapeutically effective amount") may vary depending on a variety of factors, such as the disease state, age and body weight of the patient, and the ability of the drug to produce a desired therapeutic effect in the patient. Whether a disease symptom has been alleviated can be evaluated by any clinical testing methods commonly used by doctors or other health care professionals to evaluate the severity or progression of the symptom. Although the embodiments of the present disclosure (e.g., treatment methods or products) may not be effective in alleviating every disease symptom of interest, as determined according to any statistical testing method known in the art, such as Student t-test, chi-square test, Mann and Whitney's U test, Kruskal-Wallis test (H test), Jonckheere-Terpstra test, and Wilcoxon test, they should reduce the disease symptoms of interest in a statistically significant number of patients.

"Effective amount" comprises an amount sufficient to ameliorate or prevent a symptom or disorder of a medical disease. An effective amount also means an amount sufficient to allow or facilitate diagnosis. The effective amount for a particular patient or veterinary subject may vary depending on factors such as the disorder to be treated, the general health of the patient, the method and route and dose of administration, and the severity of adverse effects. An effective amount may be the maximum dose or administration regimen to avoid significant side effects or toxic effects.

The "n" in the anti-HER2 antibody-drug conjugate of the present disclosure refers to the average number of cytotoxic drugs loaded on each antibody or an antigen-binding fragment thereof in the antibody-drug conjugate molecule, and it may also be expressed as the ratio of the number of drugs to the number of antibodies, which is the average number of drugs per ADC molecule after a coupling reaction as identified by hydrophobic interaction chromatography (HIC) or mass spectrometry.

In the present disclosure, the so-called "combination" is a mode of administration and includes various situations where two or more drugs are administered sequentially or simultaneously. The modes of administration of simultaneous administration, independent formulation and co-administration, or independent formulation and sequential administration all belong to the combination administration described in the present disclosure. Here, the so-called "simultaneous" refers to the administration of at least one dose of an anti-HER2 antibody-drug conjugate and an additional therapeutic agent within a certain period of time, e.g., administration of the two drugs within two days or one day, wherein both substances exhibit pharmacological effects. The so-called "sequential" administration includes the case where the anti-HER2 antibody-drug conjugate and the additional therapeutic agent are administered separately within different administration cycles. The period of time may be within one administration cycle, optionally within 4 weeks, within 3 weeks, within 2 weeks, within 1 week, within 24 h, or within 2 h. Such periods of time include treatment in which the anti-HER2 antibody-drug conjugate and the additional therapeutic agent are administered via the same route of administration or different routes of administration.

### DETAILED DESCRIPTION

The present disclosure is further described below with reference to examples; however, these examples are not intended to limit the scope of the present disclosure. The experimental methods in the examples of the present disclosure in which specific conditions are not specified are generally performed under conventional conditions, for example, by referring to Antibodies: A Laboratory Manual and Molecular Cloning: A Laboratory Manual by Cold Spring Harbor Laboratory, or under conditions recommended by the manufacturer of the raw material or the goods. Reagents without specific origins indicated were commercially available conventional reagents.

### Example 1. Preparation of Anti-HER2 Antibody-Drug Conjugate

According to the production method described in WO2021190581A, an anti-HER2 antibody-drug conjugate represented by the following structure was prepared using trastuzumab (an anti-HER2 antibody) and an exatecan analog, and the average was calculated by the HIC method: n = 6.0, i.e., ADC-32.

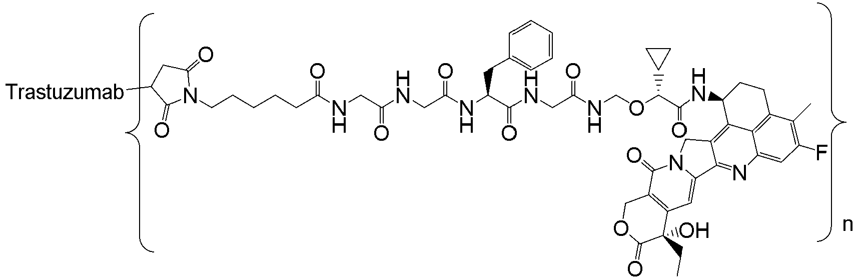

The sequences of trastuzumab are shown below:
Light chain: 
DIQMTQSPSSLSASVGDRVTITCRASQDVNTAVAWYQQKPGKAPKLLIYSASFLYSGVP
SRFSGSRSGTDFTLTISSLQPEDFATYYCQQHYTTPPTFGQGTKVEIKRTVAAPSVFIFPPS
DEQLKSGTASVVCLLNNFYPREAKVQWKVDNALQSGNSQESVTEQDSKDSTYSLSSTLTLSK
ADYEKHKVYACEVTHQGLSSPVTKSFNRGEC
SEQ ID NO: 1
Heavy chain:

EVQLVESGGGLVQPGGSLRLSCAASGFNIKDTYIHWVRQAPGKGLEWVARIYPTNGYT
RYADSVKGRFTISADTSKNTAYLQMNSLRAEDTAVYYCSRWGGDGFYAMDYWGQGT
LVTVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPELLGG
PSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSREEMTK
NQVSLTCLVKGFYPSDIAVEWESNGQPENNYKTTPPVLDSDGSFFLYSKLTVDKSRWQQGN
VFSCSVMHEALHNHYTQKSLSLSPGK
SEQ ID NO: 2

Note: The CDR sequences defined according to the Kabat numbering scheme are underlined, and the constant region sequences are italicized.

### Example 2. Clinical Study of Anti-HER2 Antibody-Drug Conjugate in Breast Cancer (BC) Patients with Low HER2 Expression

### I. Investigational drug

The anti-HER2 antibody-drug conjugate described in Example 1, freeze-dried powder, strength: 100 mg/vial.

### II. Enrolled subjects

1. Age ≥ 18 years.
2. Patients with pathologically or cytologically confirmed recurrent or metastatic breast cancer with low HER2 expression (IHC2+ and ISH negative, IHC1+ and ISH negative, or IHC1+ and ISH not tested).

### III. Mode of administration

Eligible subjects were administered the corresponding anti-HER2 antibody-drug conjugate. The anti-HER2 antibody-drug conjugate was administered at a dose of 1.0 mg/kg, 2.0 mg/kg, 3.2 mg/kg, 4.8 mg/kg, 6.4 mg/kg, or 8.0 mg/kg by an intravenous drip once every 3 weeks, with every 3 weeks (21 days) as one cycle.

### IV. Results

Some of the efficacy data are shown in Table 2. A total of 77 patients were enrolled, with an ORR of 55.8% (43/77, 95% CI 44.1-67.2) for BC patients with low HER2 expression.

**Table 2. Subgroup analysis of ORR**

| Patients' number of prior treatment lines in metastatic setting (N = 77) | |
|---|---|
| ≤3 | 58.7% (27/46) |
| >3 | 51.6% (16/31) |

| BC patients' prior anti-HER2 treatments (N = 77)* | |
|---|---|
| Any anti-HER2 treatment | 68.8% (11/16, 41.3-89.0) |
| Trastuzumab | 75.0% (9/12, 42.8-94.5) |
| Pertuzumab | 100% (5/5, 47.8-100) |
| Pyrotinib | 71.4% (5/7, 29.0-96.3) |
| Lapatinib | 100% (1/1, 2.5-100) |
| T-DM1 | 100% (3/3, 29.2-100) |
| Other HER2-ADCs (except for T-DM1) ** | 50.0% (2/4, 6.8-93.2) |

The ORR is shown in % (n/N, 95% CI) or % (n/N).
*The ORR was calculated using the number of subjects who have previously received anti-HER2 cancer treatment in the advanced/metastatic setting as a denominator; two-sided 95% CIs were estimated using the Clopper-Pearson method.
**Including RC48-ADC, A166, DP303c, MRG002, ARX788, TAA013, DX126-262, PF-06804103, and BAT8001.

Subsequently, another 110 evaluable breast cancer patients with low HER2 expression were enrolled in the clinical trial, and their efficacy evaluation results are shown in Tables 3 and 4.

**Table 3. Tumor responses in breast cancer patients with low HER2 expression receiving the anti-HER2 antibody-drug conjugate**

| Dose | 4.8 mg/kg | 5.6 mg/kg | 6.4 mg/kg | ALL |
|---|---|---|---|---|
| Number of patients, n | 37 | 33 | 37 | 110 |
| (cBOR), n (%) | | | | |
| CR | 0 | 0 | 1 (2.7) | 1 (0.9) |
| PR | 20 (54.1) | 20 (60.6) | 23 (62.2) | 64 (58.1) |
| SD | 14 (37.8) | 9 (27.3) | 11 (29.7) | 36 (32.7) |
| PD | 2 (5.4) | 2 (6.1) | 2 (5.4) | 6 (5.5) |
| NE | 1 (2.7) | 2 (6.1) | 0 | 3 (2.7) |
| cORR, n (%, 95% CI) | 20 (54.1%, 36.9-70.5) | 20 (60.6%, 42.1-77.1) | 24 (64.9%, 47.5-79.8) | 65 (59.1%, 49.3-68.4) |
| uORR, n (%, 95% CI) | 25 (67.6%, 50.2-82.0) | 21 (63.6%, 45.1-79.6) | 27 (73.0%, 55.9-86.2) | 74 (67.3%, 57.7-75.9) |

| | | | | |
|---|---|---|---|---|
| Note: The subject must have one efficacy evaluation every 6 weeks (±7 days) within the first 48 weeks after the first dose, and one evaluation every 12 weeks (±7 days) thereafter. Imaging evaluations were not affected by administration interruption or delay. Subjects with a first evaluation of CR or PR should be confirmed after 4 weeks (planned next time point). | | | | |

cBOR refers to the confirmed best overall response of the tumor, cORR refers to the confirmed objective response rate, and uORR refers to the unconfirmed objective response rate.

As can be seen from the results in Table 3, in the dose groups, the confirmed tumor objective response rate (cORR) of breast cancer patients with low HER2 expression could be up to 64.9% (administration at 6.4 mg/kg) and 60.6% (administration at 5.6 mg/kg); moreover, in all breast cancer patients with low HER2 expression receiving administration at different doses, the cORR reached 59.1%, and the uORR reached 67.3%. This indicates that the anti-HER2 antibody-drug conjugate provided in the present disclosure, when used for treating breast cancer patients with low HER2 expression, can exert a significant tumor-inhibiting effect.

**Table 4. The PFS and DoR of breast cancer patients with low HER2 expression receiving the anti-HER2 antibody-drug conjugate**

| Dose | 4.8 mg/kg | 5.6 mg/kg | 6.4 mg/kg | ALL |
|---|---|---|---|---|
| Number of patients, n | 37 | 33 | 37 | 110 |
| PFS Event, n (%) | 24 (64.9) | 9 (27.3) | 17 (45.9) | 53 (48.2) |
| mPFS, mo (95% CI) | 8.2 (5.5-13.6) | NE (5.7-NE) | 13.8 (9.6-NE) | 10.9 (7.9-13.8) |
| 06-mo PFS rate, % | 63.1 | 66.8 | 85.2 | 71.2 |
| 12-mo PFS rate, % | 34.7 | NE | 57 | 43.9 |
| 18-mo PFS rate, % | 27.6 | NE | 41.7 | 33.6 |
| mDoR, mo (95% CI) | 9.9 (5.0-NE) | NE (4.5-NE) | 16.7 (7.2-NE) | 12.2 (7.2-NE) |
| 06-mo DoR rate, % | 72.7 | 66.1 | 78.2 | 73.3 |
| 12-mo DoR rate, % | 55.3 | NE | 59 | 51.6 |

| | | | | |
|---|---|---|---|---|
| Note: PFS refers to progression-free survival, mPFS refers to median progression-free survival, 06-mo PFS rate refers to the incidence of progression-free survival at 6 months, 12-mo PFS rate refers to the incidence of progression-free survival at 12 months, and 18-mo PFS rate refers to the incidence of progression-free survival at 18 months. mDoR refers to the median duration of response. | | | | |

As can be seen from the results in Table 4, in breast cancer patients with low HER2 expression treated with the anti-HER2 antibody-drug conjugate, the median progression-free survival (mPFS) could be up to 13.8 months (at a dose of 6.4 mg/kg), and the median duration of response (mDoR) could be up to 16.7 months (at a dose of 6.4 mg/kg). Moreover, in 110 breast cancer patients with low HER2 expression who received treatment with the anti-HER2 antibody-drug conjugate, the median progression-free survival (mPFS) could be up to 10.9 months, and the median duration of response (mDoR) could be up to 12.2 months. This indicates that the anti-HER2 antibody-drug conjugate provided in the present disclosure, when used for treating breast cancer patients with low HER2 expression, can significantly increase the patients' progression-free survival time and the duration of response of the tumor, so that the breast cancer patients with low HER2 expression can be effectively treated.

### Example 3. Phase I Clinical Study of Anti-HER2 Antibody-Drug Conjugate

In the phase I clinical study of the anti-HER2 antibody-drug conjugate, treatment-related adverse events (TRAEs) were reported in 243 patients with advanced solid tumors (97.2%). Grade ≥3 TRAEs, severe TRAEs, and treatment-related deaths were reported in 131 (52.4%), 31 (12.4%), and 3 (1.2%) patients, respectively. Interstitial lung disease (AESI) was reported in 8 (3.2%) subjects. The exposure of the anti-HER2 antibody-drug conjugate, the total antibody, and the payload was generally directly proportional to the dose of 3.2 to 8.0 mg/kg. The ORR for all patients was 61.6% (154/250, 95% CI 55.3-67.7).

### Example 4. Open, Multi-Center Phase Ib/II Clinical Study of Combinations of Anti-HER2 Antibody-Drug Conjugate with Isethionate of Compound Represented by Formula (II), Fulvestrant, Bevacizumab, and Letrozole/Anastrozole for Treatment of Unresectable or Metastatic Breast Cancer with Low HER2 Expression

### I. Investigational drugs

The anti-HER2 antibody-drug conjugate described in Example 1, freeze-dried powder, strength: 100 mg/vial.

The isethionate of the compound represented by formula (II), tablets, strength: 25 mg/tablet, 50 mg/tablet, 125 mg/tablet, and 150 mg/tablet.

Fulvestrant injection (Pulaihe), injection, strength: 5 mL: 0.25 g.

Letrozole tablets (Furui), tablets, strength: 2.5 mg.

Anastrozole tablets (Yishuzhi), tablets, strength: 1 mg.

Bevacizumab injection (Airuituo), injection, strength: 100 mg (4 mL)/vial.

### II. Enrolled subjects

1. Female aged 18 to 75 years (inclusive).
2. Histologically or cytologically confirmed unresectable or metastatic breast cancer with low HER2 expression (IHC2+/ISH-, IHC1+/ISH- or not tested); the estrogen receptor/progestin receptor (ER/PR) status should be determined in the recurrent/metastatic stage [the definition of ER-positive and/or PR-positive should meet the American Society of Clinical Oncology/College of American Pathologists (ASCO/CAP) guidelines]. The low HER2 expression should be verified and confirmed by the pathology department of the research participation center of the study.
3. HR-positive subjects should meet any one of the following:
   a) previously received bilateral ovariectomy, or age ≥ 60 years; or
   b) age < 60, in a natural postmenopausal state (defined as at least 12 consecutive months of spontaneous cessation of regular menstruation without other pathological or physiological causes), estradiol (E2) and follicle-stimulating hormone (FSH) at postmenopausal levels; or
   c) premenopausal or perimenopausal female patients can also be included, but they must be willing to receive luteinizing hormone-releasing hormone (LHRH) agonist treatment during the study.
4. Evidence of imaged or objective disease progression during or after the last systemic treatment prior to starting study treatment.
5. Prior treatments:
   HR-positive subjects:
      a) Phase one (dose finding): The subjects have received at least first-line endocrine therapy, and ≤ second-line chemotherapy is allowed.
      b) Phase two (efficacy expansion): The subjects are allowed to have received ≤ first-line endocrine therapy, and have not received chemotherapy in the recurrent/metastatic stage.
   HR-negative subjects:
      c) Phase one (dose finding): The subjects have received at least first-line chemotherapy.
      d) Phase two (efficacy expansion): The subjects have not received systemic anti-tumor treatment in the recurrent/metastatic stage.
      e) Disease recurrence within the first 24 months after adjuvant endocrine therapy is considered as one-line treatment; recurrence within 6 months after (neo)adjuvant chemotherapy is counted as a first-line chemotherapy regimen.

### III. Mode of administration

Eligible subjects are administered corresponding drugs.

Phase one (dose finding phase):
The dose finding phase is intended to evaluate the safety, tolerability, pharmacokinetic characteristics, and immunogenicity of combinations of the anti-HER2 antibody-drug conjugate with the isethionate of the compound represented by formula (II), fulvestrant, and bevacizumab, and to preliminarily observe their anti-tumor efficacy.

### Combination of anti-HER2 antibody-drug conjugate and isethionate of compound represented by formula (II):

The anti-HER2 antibody-drug conjugate is administered at a dose of 2.0 mg/kg, 3.2 mg/kg, or 4.8 mg/kg by an intravenous drip once every 3 weeks, with every 3 weeks (21 days) as one cycle.

The isethionate of the compound represented by formula (II) is orally administered at a dose of 100 mg, 125 mg, or 150 mg once daily, with 2 weeks of treatment/1 week of interruption as a course of treatment.

If a related dose group is intolerable, the administration cycle, administration time sequence, and usage and dosage of the study drug can be adjusted, including but not limited to: a. adjusting the administration cycle of the anti-HER2 antibody-drug conjugate to once every 2 weeks and correspondingly adjusting the dose; b. adjusting the course of treatment of the isethionate of the compound represented by formula (II) to 3 weeks of treatment/1 week of interruption; c. adjusting the administration time sequence of the anti-HER2 antibody-drug conjugate and the isethionate of the compound represented by formula (II), for example, administering at intervals of several days, etc.

### Combination of anti-HER2 antibody-drug conjugate and fulvestrant:

The anti-HER2 antibody-drug conjugate is administered at a dose of 2.0 mg/kg, 3.2 mg/kg, or 4.8 mg/kg once every 3 weeks.

Fulvestrant is administered at a dose of 500 mg, with every 4 weeks as one course of treatment. Administration was performed on days 1 and 15 of the first course of treatment and day 1 of every subsequent course of treatment.

If a related dose group is intolerable, the administration cycle, administration time sequence, and usage and dosage of the study drug can be adjusted, including but not limited to: adjusting the administration cycle of the anti-HER2 antibody-drug conjugate to once every 2 weeks and correspondingly adjusting the dose, etc.

### Combination of anti-HER2 antibody-drug conjugate and bevacizumab:

The anti-HER2 antibody-drug conjugate is administered at a dose of 2.0 mg/kg, 3.2 mg/kg, or 4.8 mg/kg once every 3 weeks.

Bevacizumab is administered at a fixed dose of 15 mg/kg once every 3 weeks.

If a related dose group is intolerable, the administration cycle, administration time sequence, and usage and dosage of the study drug can be adjusted, including but not limited to: adjusting the administration cycle of the anti-HER2 antibody-drug conjugate to once every 2 weeks and correspondingly adjusting the dose, etc.

### Phase two (efficacy expansion phase):

The efficacy expansion phase is intended to observe and evaluate the preliminary efficacy, safety, pharmacokinetic characteristics, and immunogenicity of combinations of the anti-HER2 antibody-drug conjugate with the isethionate of the compound represented by formula (II) and letrozole/anastrozole, fulvestrant, letrozole/anastrozole, and bevacizumab.

### Combination of anti-HER2 antibody-drug conjugate and isethionate of compound represented by formula (II) with letrozole/anastrozole:

After the dose finding of phase one is completed for the combination of the anti-HER2 antibody-drug conjugate and the isethionate of the compound represented by formula (II), at least one dose group of the anti-HER2 antibody-drug conjugate and the isethionate of the compound represented by formula (II) is selected for efficacy expansion in combination with letrozole or anastrozole.

Letrozole is continuously orally administered once daily, 2.5 mg each time.

Anastrozole is continuously orally administered once daily, 1 mg each time.

### Combination of anti-HER2 antibody-drug conjugate and fulvestrant:

After the dose finding of phase one is completed for the combination of the anti-HER2 antibody-drug conjugate and fulvestrant, 1-2 doses are selected for efficacy expansion. Eligible subjects will enter the combination of the anti-HER2 antibody-drug conjugate and fulvestrant for efficacy expansion.

### Combination of anti-HER2 antibody-drug conjugate and letrozole/anastrozole:

After the dose finding of phase one is completed for the combination of the anti-HER2 antibody-drug conjugate and fulvestrant, 1-2 doses will be selected for efficacy expansion of the anti-HER2 antibody-drug conjugate and letrozole/anastrozole with reference to the safety of the dose group(s).

Letrozole is continuously orally administered once daily, 2.5 mg each time.

Anastrozole is continuously orally administered once daily, 1 mg each time.

### Combination of anti-HER2 antibody-drug conjugate and bevacizumab:

After the dose finding of phase one is completed for the combination of the anti-HER2 antibody-drug conjugate and bevacizumab, at least one dose group is selected for efficacy expansion.

## Claims

1. Use of an anti-HER2 antibody-drug conjugate in the manufacture of a medicament for treating breast cancer with low HER2 expression, wherein the antibody-drug conjugate has a structure represented by formula (I): 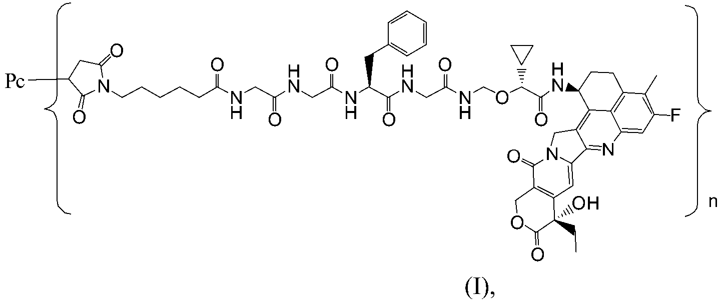 wherein:
n is a decimal or integer from 3 to 8;
Pc is an anti-HER2 antibody.

2. The use according to claim 1, wherein the anti-HER2 antibody is selected from the group consisting of trastuzumab, pertuzumab, and antigen-binding fragments thereof; preferably, the anti-HER2 antibody is trastuzumab or an antigen-binding fragment thereof.

3. The use according to claim 1 or 2, wherein the anti-HER2 antibody comprises a heavy chain variable region and a light chain variable region, wherein the heavy chain variable region comprises a HCDR1, a HCDR2, and a HCDR3 set forth in SEQ ID NOs: 3, 4, and 5, respectively, and a LCDR1, a LCDR2, and a LCDR3 set forth in SEQ ID NOs: 6, 7, and 8, respectively;
preferably, the heavy chain variable region comprises the amino acid sequence set forth in SEQ ID NO: 9 or an amino acid sequence having at least 90% identity thereto, and the light chain variable region comprises the amino acid sequence set forth in SEQ ID NO: 10 or an amino acid sequence having at least 90% identity thereto;
preferably, the anti-HER2 antibody comprises a heavy chain and a light chain, wherein the heavy chain comprises the amino acid sequence set forth in SEQ ID NO: 2 or an amino acid sequence having at least 90% identity thereto, and the light chain comprises the amino acid sequence set forth in SEQ ID NO: 1 or an amino acid sequence having at least 90% identity thereto.

4. The use according to any one of claims 1-3, wherein the anti-HER2 antibody-drug conjugate has a structure represented by the following formula: 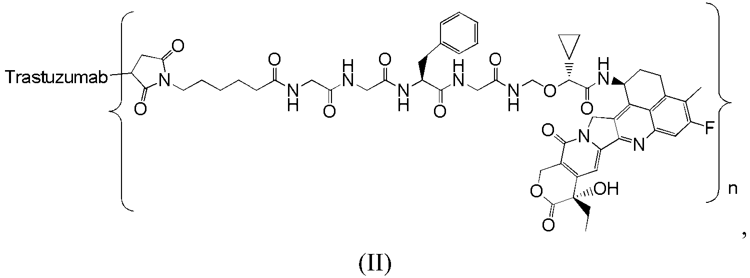 wherein n is a decimal or integer from 3 to 8, preferably 6 ± 0.8.

5. The use according to any one of claims 1-4, wherein the breast cancer with low HER2 expression is breast cancer with HER2 expression determined to be 1+ by immunohistochemistry, or
breast cancer with HER2 expression determined to be 2+ by immunohistochemistry and to be negative by *in situ* hybridization.

6. The use according to any one of claims 1-5, wherein the breast cancer with low HER2 expression is unresectable, recurrent, and/or metastatic breast cancer with low HER2 expression.

7. The use according to any one of claims 1-6, wherein the breast cancer with low HER2 expression has previously received treatment with an anti-HER2 drug; preferably, the breast cancer with low HER2 expression develops resistance or refractoriness after having previously received treatment with an anti-HER2 drug.

8. The use according to claim 7, wherein the anti-HER2 drug is at least one drug selected from the group consisting of trastuzumab, pertuzumab, pyrotinib, lapatinib, and T-DM1.

9. The use according to any one of claims 1-8, wherein the use is use of the anti-HER2 antibody-drug conjugate in combination with a second therapeutic agent in the manufacture of a medicament for treating breast cancer with low HER2 expression.

10. The use according to claim 9, wherein the second therapeutic agent is a CDK4/6 inhibitor; preferably, the CDK4/6 inhibitor is selected from the group consisting of abemaciclib, ribociclib, palbociclib, alvocidib, trilaciclib, voruciclib, AT-7519, G1T-38, FLX-925, INOC-005, G1T28-1, BPI-1178, gossypin, G1T30-1, GZ-38-1, P-276-00, staurosporine, R-547, PAN-1215, PD-0183812, AG-024322, NSC-625987, CGP-82996, PD-171851, and the compound represented by formula (II) or a pharmaceutically acceptable salt thereof; more preferably, the CDK4/6 inhibitor is the compound represented by formula (II) or a pharmaceutically acceptable salt thereof; most preferably, the CDK4/6 inhibitor is isethionate of the compound represented by formula (II), 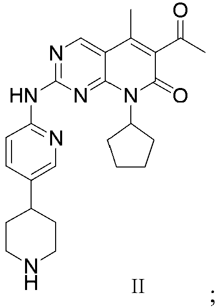
preferably, the CDK4/6 inhibitor is administered at a dose of 1-1000 mg, preferably 100 mg, 125 mg, or 150 mg, and a preferred frequency of administration may be once daily, twice daily, three times daily, once weekly, once every two weeks, once every three weeks, or once monthly;
preferably, the anti-HER2 antibody-drug conjugate is administered once every two weeks or once every three weeks; the CDK4/6 inhibitor is administered once daily, with continuous administration for the first 2 weeks and interruption for the following week, or continuous administration for the first 3 weeks and interruption for the following week.

11. The use according to claim 9, wherein the second therapeutic agent is an aromatase inhibitor; preferably, the aromatase inhibitor is selected from the group consisting of formestane, exemestane, fadrozole, letrozole, vorozole, and anastrozole; more preferably, the aromatase inhibitor is letrozole or anastrozole;
preferably, the aromatase inhibitor is administered at a dose of 0.1-50 mg, preferably 0.5 mg, 1.0 mg, 1.5 mg, 2.0 mg, 2.5 mg, 3.0 mg, 3.5 mg, 4.0 mg, 4.5 mg, or 5.0 mg, and a preferred frequency of administration is once daily or twice daily;
preferably, the anti-HER2 antibody-drug conjugate is administered once every two weeks or once every three weeks; the aromatase inhibitor is continuously administered once daily.

12. The use according to any one of claims 9-11, wherein the second therapeutic agent is a CDK4/6 inhibitor and an aromatase inhibitor.

13. The use according to claim 9, wherein the second therapeutic agent is a SERD; preferably, the SERD is fulvestrant, AZD-9496, RAD1901, or ZB-716; more preferably, the SERD is fulvestrant;
preferably, the SERD is administered at a dose of 1-1000 mg, preferably 500 mg, and a preferred frequency of administration is once daily, twice daily, three times daily, once weekly, once every two weeks, once every three weeks, or once monthly;
preferably, the anti-HER2 antibody-drug conjugate is administered once every two weeks or once every three weeks; the SERD is administered with every 4 weeks as one treatment cycle, wherein administration is performed on days 1 and 15 of the first cycle and day 1 of every subsequent cycle.

14. The use according to claim 9, wherein the second therapeutic agent is a VEGF ligand inhibitor; preferably, the VEGF ligand inhibitor is selected from the group consisting of bevacizumab, ramucirumab, ranibizumab, aflibercept, conbercept, abicipar pegol, brolucizumab, LMG-324, nesvacumab, sevacizumab, tanibirumab, navicixizumab, RG-7716, LHA-510, OPT-302, TK-001, GZ-402663, VGX-100, PG-545, BI-836880, GNR-011, BR-55, OTSGC-A24, PAN-90806, AVA-101, ODM-203, TAS-115, X-82, MP-0250, sitravatinib, 4SC-203, AL-2846, ABT-165, SIM-010603, BI-836880, HL-217, CS-2164, RGX-314, AMC-303, and VXM-01; more preferably, the VEGF ligand inhibitor is bevacizumab;
preferably, the VEGF ligand inhibitor is administered at a dose of 0.1-100 mg/kg, preferably 15 mg/kg, and a preferred frequency of administration is once daily, once weekly, once every two weeks, once every three weeks, or once monthly;
preferably, the anti-HER2 antibody-drug conjugate is administered once every two weeks or once every three weeks; the VEGF ligand inhibitor is administered once every three weeks.

15. The use according to any one of claims 1-14, wherein a single-time administration dose of the anti-HER2 antibody-drug conjugate is 1.0 mg/kg-10.0 mg/kg, preferably 1.0 mg/kg, 2.0 mg/kg, 3.2 mg/kg, 4.8 mg/kg, 5.6 mg/kg, 6.4 mg/kg, or 8.0 mg/kg, and a preferred frequency of administration is once every week, once every two weeks, once every three weeks, or once every four weeks.

16. A method for treating breast cancer with low HER2 expression, comprising administering to a subject in need thereof an anti-HER2 antibody-drug conjugate, wherein the anti-HER2 antibody-drug conjugate has a structure represented by formula (I): 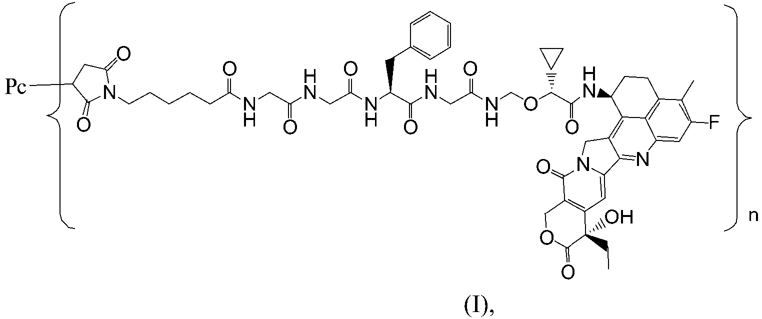 wherein:
n is a decimal or integer from 3 to 8;
Pc is an anti-HER2 antibody; the anti-HER2 antibody-drug conjugate is as defined in any one of claims 1-4 and 15; the breast cancer with low HER2 expression is as defined in any one of claims 5-8;
preferably, the method comprises administering to a subject in need thereof an anti-HER2 antibody-drug conjugate and a second therapeutic agent, wherein the second therapeutic agent is one or more agents selected from the group consisting of a CDK4/6 inhibitor, a SERD, a VEGF ligand inhibitor, and an aromatase inhibitor.

17. A pharmaceutical composition, comprising an anti-HER2 antibody-drug conjugate and a second therapeutic agent, and one or more pharmaceutically acceptable carriers, wherein the anti-HER2 antibody-drug conjugate has a structure represented by formula (I): 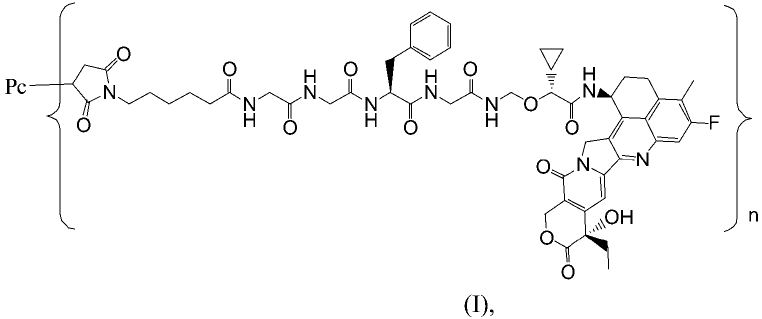 wherein:
n is a decimal or integer from 3 to 8;
Pc is an anti-HER2 antibody; the anti-HER2 antibody-drug conjugate is preferably as defined in any one of claims 1-4 and 15; the second therapeutic agent is preferably as defined in any one of claims 9-14.
